# EUROPEAN PATENT APPLICATION

(11) **EP 4 001 282 A1**
(43) Date of publication of application: **25.05.2022**
(21) Application number: 20839690.3
(22) Date of filing: 14.07.2020
(51) Int. Cl.: C07D 498/04, A61K 31/4985, A61P 35/00

(54) **STEREOISOMERS OF THE COMPOUND 3-(BENZO[D][1,3]DIOXOL-5-YL)-7-(1-HYDROXYPROPAN-2-YL)-1-(1H-INDOL-3-YL)-6,7-DIHYDRO-3H-OXAZOL[3,4-A]PYRAZINE-5,8-DIONE AND USE THEREOF AS AN ANTITUMOR AGENT AND PHOSPHODIESTERASE ENZYME INHIBITOR**

(30) Priority: 15.07.2019 US 201962874321 P
(71) Applicant: Biolab Sanus Farmacéutica Ltda, 06767-220 Taboão da Serra - SP (BR)
(72) Inventor: SACURAI, Sergio Luiz, 04052-020 São Paulo - SP (BR); DA COSTA TOUZARIM, Carlos Eduardo, 05783-070 São Paulo - SP (BR); TOLEDO, Fabiano Travanca, 06213-130 Osasco - SP (BR); DOS SANTOS FERRARINI, Renan, 05360-030 São Paulo - SP (BR); HELFSTEIN, De bora Rocha, 04775-180 São Paulo - SP (BR); ZAMINELLI, Tiago, 05345-000 São Paulo - SP (BR); MOSCOSO, Julio Alejandro Rojas, 13208-160 Jundiaí - SP (BR); DAS VIRGENS, Marcio Fernando, 09991-000 Diadema - SP (BR)
(74) Representative: HGF
(86) International application number: PCT/BR2020/050259
(87) International publication number: WO 2021/007636

(57) **Abstract**

The present invention relates to the compounds 3-(benzo[d][1,3]dioxol-5-yl)-7-(1-hydroxypropan-2-yl)-1-(1*H-*indol-3-yl)-6,7-dihydro-3H-oxazol[3,4-a]pyrazine-5,8-dione of formula (I)), 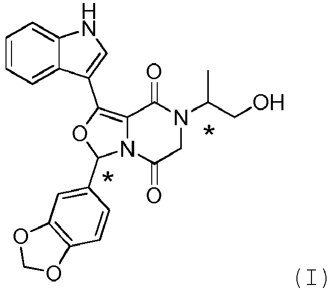 and also to pharmaceutically acceptable stereoisomers, salts, solvates, hydrates, prodrugs and esters thereof; the stereoisomers being in their separate individual forms and/or in the forms of racemic mixtures or non-racemic mixtures with diastereomeric excess in any proportions, a pharmaceutical composition comprising at least one of the compounds described; the use of said stereoisomers as an antitumor agent or phosphodiesterase enzyme inhibitor, and the use of said stereoisomers in the treatment of benign prostatic hyperplasia and cancer, more specifically prostate cancer.

## Description

### Technology Field

The present patent application refers, in a first aspect, to compounds which are phosphodiesterase enzyme inhibitors (PDE).

The present application refers, in a second aspect, to antiproliferative and antitumor compounds.

Said compounds can be used, for example, as anti-inflammatory, vasodilators, stimulants and inotropic in cardiac insufficiency and pulmonary diseases. Notably, the PDE inhibitors have showed themselves to be useful in the treatment of erectile dysfunction in men. Additionally, said compounds can be used as antiproliferative and antitumor.

### State of the Art

The phosphodiesterase inhibitor compounds are the main agents used in medical clinic for the treatment and/or prevention of erectile dysfunction, disorders and/or conditions that are treatable with tissue relaxation and other diseases that are treatable with phosphodiesterase inhibitors. In the case of erectile dysfunction, the PDE-5 inhibitors are the ones most used in medical clinic.

The erectile dysfunction, more commonly known as sexual impotence is one of the diseases which is most harmful to the quality of life of a man. For a long time, the erectile dysfunction haunted men without much possibility of effective treatment.

Before the 70s, nearly all the erectile dysfunction cases were considered as resulting from psychological causes, and the treatments consisted in the empirical administration of testosterone or referral to a psychiatrist.

The oral treatment arose from clinical research on the use of cGMP-PDE inhibitors, more specifically, the PDE-5 inhibitors. The precursor of these compounds was the 5-[2-ethoxy-5-(4-methylpiperazinyl-sulfonyl) phenyl] -1-methyl-3-n-propyl-1, 6-dihydro-7H-pyrazole [4,3-d] pyrimidine-7-one, or sildenafil, with vasodilator properties and which potentializes the effects of the nitric oxide. The sildenafil molecule was originally described in North-American patent US5250534.

Subsequently, other inhibitor compounds of the PDE-5 enzyme were developed and are cited in a large number of technical literature publications, as well as in patent publications. Among the known compounds we highlight: the vardenafil molecule, originally described in patent US3635178; the tadalafil, originally described in American patent US5859006; The BL-106, BL-230 and BL-236 compounds which are not commercialized yet, originally described in American patents US8338432 and US9359378 and developed by the same research team as the present patent application.

Additionally, the researchers verified that the tissue relaxant compounds have been used to promote the relaxation of several tissues with the purpose of treating or acting as adjuvant in the treatment, procedure or surgery related to lithiasis (Korkes, F. et al, J Bras Nefrol. (2009) 31(1):55), prostate enlargement (for example, prostatic hyperplasia, prostatitis) (WO 9911279) and urethral constriction (Van der werf et al, BJU International (2002) 90:588). Other tissue relaxant treatable conditions and/or disorders are known and described in the state of the art.

In view of this scenario and seeking new alternatives of phosphodiesterase inhibitor compounds, more specifically as PDE-5 inhibitors, for the treatment of erectile dysfunction, tissue relaxant treatable conditions and/or disorders and other diseases that are treatable with phosphodiesterase inhibitors, the researchers of the present invention developed new compounds which are now described and claimed, comprising the compound 3- (benzo [d] [1 , 3] dioxol-5-yl) -7- (1-hydroxypropan-2-yl) -1- (1H-indole-3-yl) -6, 7-dihydro-3H-oxazole [3, 4-a] pyrazine-5, 8-dione and the stereoisomers thereof which presented, together and separately, prominent activities as PDE inhibitors, antiproliferative and antitumor.

In this manner, under one aspect, the compounds now claimed are presented as alternatives for the treatment of several diseases and medical conditions which benefit from the PDE inhibition, such as erectile dysfunction, cardiac insufficiency, pulmonary hypertension and for the treatment of several medical diseases and conditions which benefit from the antiproliferative and antitumor effects.

### Detailed description

The present patent application refers, in one aspect to the compound 3-(benzo[d] [1,3] dioxol-5-yl)-7-(1-hydroxypropan-2-yl)-1-(1*h*-indole-3-yl)-6,7-dihydro-3*h*-oxazole[3,4-a] pyrazine-5,8-dione of formula (I) and the stereoisomers thereof, 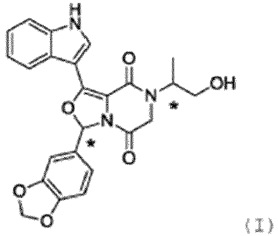
as well as their salts, solvates, hydrates, prodrugs and pharmaceutically acceptable esters;
In alternative aspects, the referred stereoisomers of the compound of formula (I) are in their individual separate forms and/or in the forms of racemic mixtures or non-racemic mixtures with diastereoisomeric excess in any proportions.

In specific aspects, the stereoisomers are the compounds of formulas (II) and (III)

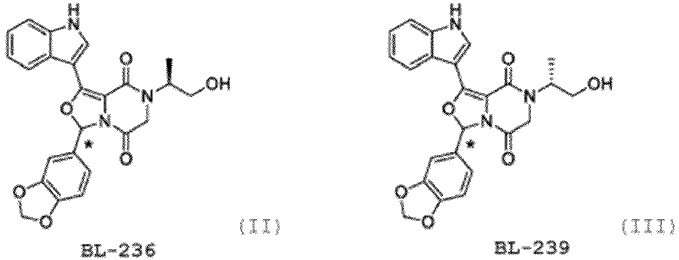

The compound of **formula (II)** is named 3-(benzo[d][1,3] dioxol-5-yl)-7-((S)1-hydroxypropan-2-yl)-1-(1*H*-indole-3-yl)-6,7-dihydro-3*H*-oxazole[3,4-a]pyrazine-5,8-dione (**BL-236**) and the compound of **formula (III)** is named 3- (benzo [d] [1,3] dioxol-5-yl) -7- ((R) 1-hydroxypropan-2-yl) -1-(1H- indole-3-yl) - 6, 7-dihydro-3H-oxazole [3, 4-a]pyrazine-5, 8-dione (**BL-239**).

In a preferred aspect, the present patent application refers to the isolated stereoisomers known by the formulas (IV), (V), (VI) and (VII), below:

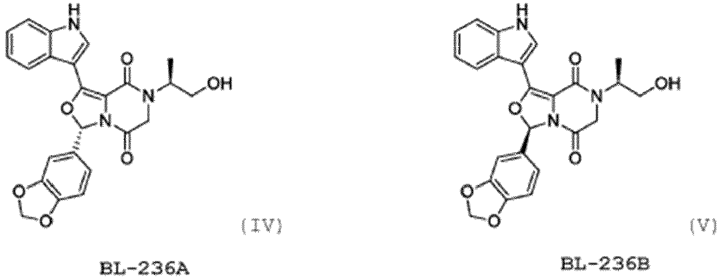

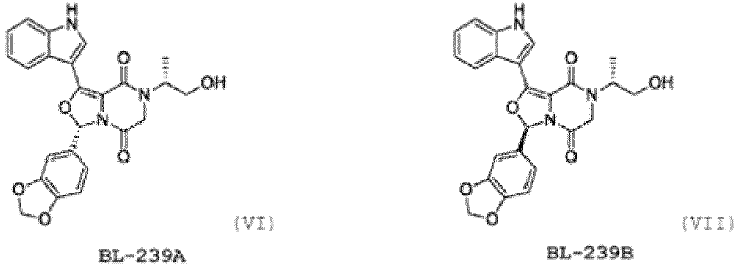

The stereoisomers described above are named:
Compound of **formula (IV):** (S) -3- (benzo [d] [1, 3] dioxol-5- yl) -7- ((S) -1-hydroxypropan-2-yl) -1-(1*H-*indole-3-yl) -6, 7- dihydro-3*H*-oxazole [3, 4-a] pyrazine-5, 8-dione (**BL-236A**);
Compound of f**ormula (V):** (R) -3- (benzo [d] [1, 3] dioxol-5- yl) -7- ((S) -1-hydroxypropan-2-yl) -1-(1*H-*indole-3-yl) -6, 7- dihydro-3*H*-oxazole [3, 4-a] pyrazine-5, 8-dione (**BL-236B**);
Compound of **formula (VI):** (S) -3- (benzo [d] [1, 3] dioxol-5- yl) -7- ((R) -1-hydroxypropan-2-yl) -1-(1*H-*indole-3-yl -6, 7-dihydro-3*H*-oxazole [3, 4-a] pyrazine-5, 8-dione (**BL-239A**); and
Compound of **formula (VII) :** (R) -3- (benzo [d] [1, 3 ] dioxol-5- yl) -7- ((R) -1-hydroxypropan-2-yl) -1- (1*H-*indole-3-yl) -6, 7- dihydro-3*H*-oxazole [3, 4-a] pyrazine-5, 8-dione (**BL-239B**).

Another aspect of the present patent application refers to mixtures of stereoisomers of the compounds 3- (benzo [d] [1,3] dioxol-5-yl) -7- (1-hydroxypropan-2-yl) -1- (1*H*- indole-3-yl) - 6, 7-dihydro-3*H-*oxazole [3, 4-a] pyrazine-5, 8-dione of formula (I), wherein the mixtures can be a racemic mixture or a non-racemic mixture.

Preferably, the present patent application seeks protection for non-racemic mixtures with diastereoisomeric excess in any proportions.

More specifically, the diastereoisomeric proportion is in the range from 1:99 to 99:1. In another preferred aspect, the mixture is in the range from 1:50 to 50:1, alternatively in the range from 1:25 to 25:1. In a more preferred aspect, the non-racemic mixtures with diastereoisomeric excess are in the range from 1:2 to 2:1, alternatively in the proportion of 1:1.

In another preferred aspect, the present patent application refers to a mixture of the diastereoisomerics described in formulas (VIII) and (IX), whereby the mixture can contain any proportions of the diastereoisomerics.

The mixture of diastereoisomerics of formula (VIII) is named (R) -3- (benzo [d] [1, 3] dioxol-5-yl) -7-((R) -1-hydroxypropan-2-yl) -1- (1*H*-indole-3-yl) -6, 7-dihydro-3H-oxazole [3, 4-a] pyrazine-5, 8-dione **with** (S)-3-(benzo [ d] [1,3] dioxol-5-yl ) -7- ((R) -1-hydroxypropan-2-yl) -1-(1*H*-indole-3-yl) - 6, 7-dihydro-3H-oxazole [3, 4-a]pyrazine-5, 8-dione (BL-380) and presents the following structure:

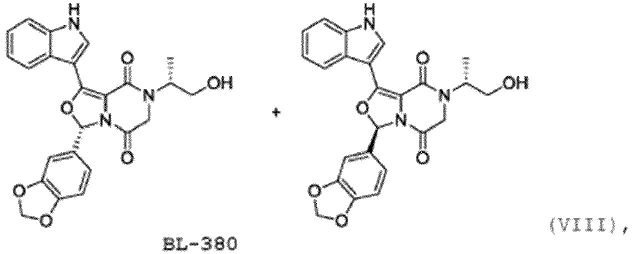

Wherein the diastereoisomeric proportion is in the range from 1:99 to 99:1. In another preferred aspect, the mixture is in the range from 1:50 to 50:1, more preferably, in the range from 1:25 to 25:1. In a more preferred aspect, the mixture is in the range from 1:2 to 2:1, more preferably in the 1:1 proportion.

The mixture of diastereoisomerics of **formula (IX)** is named (R) -3- (benzo [d] [1, 3] dioxol-5-yl) -7- ( (S) -1-hydroxypropan-2-yl) -1- (1*H*-indole-3-yl) -6, 7-dihydro-3H-oxazole [3, 4-a] pyrazine-5, 8-dione **with** (S)-3-(benzo [d] [1,3] dioxol-5-yl ) -7- ((S) -1-hydroxypropan-2-yl) -1-(1*H*-indole-3-yl) - 6, 7-dihydro-3H-oxazole [3, 4-a]pyrazine-5, 8-dione (**BL-241**), and presents the following structure: 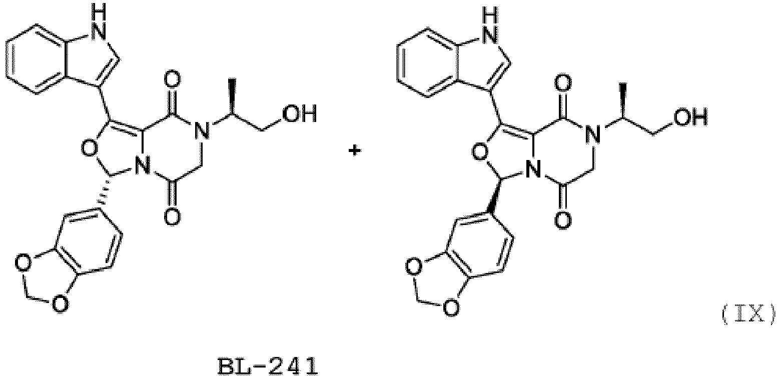 wherein the diastereoisomeric proportion is in the range from 1:99 to 99:1. In another preferred aspect, the mixture is in the range from 1:50 to 50:1, alternatively in the range from 1:25 to 25:1. In a more preferred aspect, the mixture is in the range from 1:2 to 2:1, alternatively in the 1:1 proportion.

The diastereoisomerics and mixtures thereof, which are the object of the present patent application, can be prepared, for example, according to the following reaction schemes:

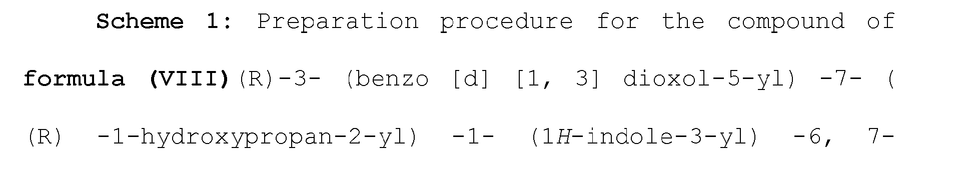

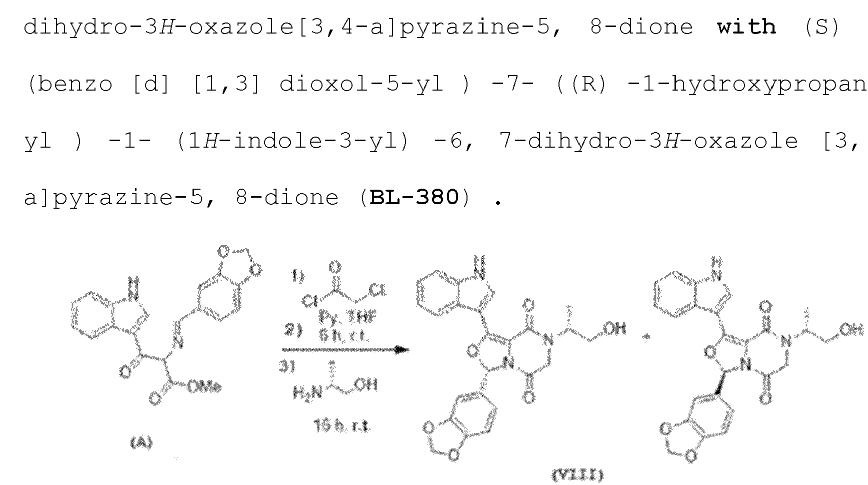

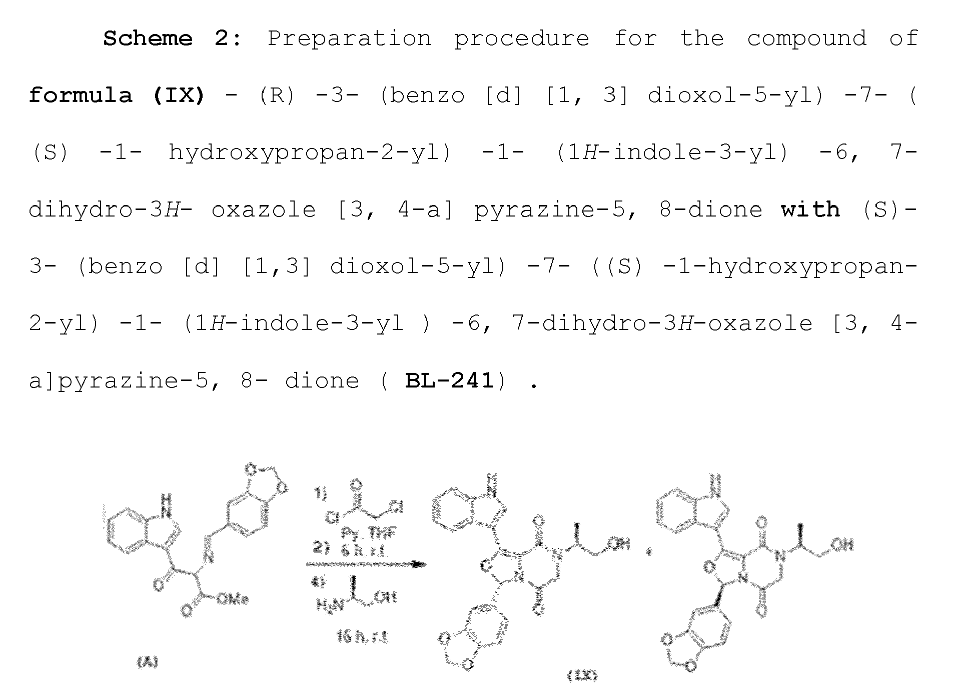

The present patent application further covers the pharmaceutical compositions comprising the referred stereoisomers of the compound of formula (I) in their individual separate forms and/or in the forms of racemic mixtures or non-racemic mixtures with stereoisomer excess in any proportions and drugs comprising the referred stereoisomers. Said compositions can be prepared according to methods that are known in the pharmaceutical technology, using thinners, excipients or pharmaceutically acceptable carriers.

Another aspect of the present patent application refers to the use of the compound of formula (I) and the stereoisomers thereof such as antitumor and phosphodiesterase enzyme inhibitors of the type 5 (PDE-5); and to the use of the referred stereoisomers in the treatment of benign prostatic hyperplasia and cancer, more specifically, prostate cancer.

In another preferred aspect the present patent application refers to the use of a mixture defined by the formula (VIII) and named as (R) -3- (benzo [d] [1 , 3] dioxol-5-yl) -7- ((R) -1-hydroxypropan-2-yl) -1- (1*H*-indole-3-yl) -6, 7-dihydro-3H-oxazole [3, 4-a] pyrazine-5, 8-dione **with** (S)-3- (benzo [d] [1,3] dioxol-5-yl ) -7- ((R) -1-hydroxypropan-2-yl) -1- (1*H*-indole-3-yl) -6, 7-dihydro-3H-oxazole [3, 4-a]pyrazine-5, 8-dione (BL-380), in any proportion of the diastereoisomers, as antitumor agent and for the treatment of disorders that are treatable with PDE-5 inhibitors in mammals. Particularly for the treatment of prostate cancer and benign prostatic hyperplasia.

Another preferred aspect of the present patent application refers to the use of isolated stereoisomers defined by the formulas (IV), (V), (VI) and (VII) and named, respectively, as: (S) -3- (benzo [d] [1 , 3] dioxol-5-yl) -7- ((S) -1-hydroxypropan-2-yl) - 1- (1*H-*indole-3-yl) - 6, 7-dihydro-3H-oxazole [3, 4-a] pyrazine-5, 8-dione (**BL-236A**) ; (R)-3- (benzo [d] [1,3] dioxol-5-yl) - 7- ((S) -1-hydroxypropan-2-yl) -1- (1*H*-indole-3-yl) - 6, 7-dihydro-3H-oxazole [3, 4-a]pyrazine-5, 8-dione (**BL-236B**) ; (S) -3- (benzo [d] [1 , 3] dioxol-5-yl) -7- ((R) -1-hydroxypropan-2-yl) -1- (1*H*-indole-3-yl) -6, 7-dihydro-3H-oxazole [3, 4-a] pyrazine-5, 8-dione (**BL-239A**) ; and (R) - 3- (benzo [d] [1,3] dioxol-5-yl) -7- ((R) -1-hydroxypropan-2-yl) -1- (1*H*-indole-3-yl) - 6, 7-dihydro-3H-oxazole [3, 4-a]pyrazine-5, 8-dione (**BL-239B**), as antitumor agent and for the treatment of disorders that are treatable with PDE-5 inhibitors in mammals. In particular for the treatment of prostate cancer and benign prostatic hyperplasia.

Additional objects of the present patent application are the uses of the compound of formula (I) and the stereoisomers thereof, salts, esters, prodrugs, as herein described, in the preparation of drugs antiproliferative, antitumor and phosphodiesterase enzyme inhibitors, more specifically inhibitors of the type 5 phosphodiesterase enzyme (PDE-5); and to the use of the referred stereoisomers in the preparation of medicament for the treatment of diseases and disorders, such as inflammatory diseases and disorders, cardiovascular, pulmonary, erectile dysfunction in man, benign prostatic hyperplasia and cancer, more specifically, prostate cancer.

The present patent application further describes a method for treating cancer, such as benign prostatic hyperplasia and cancer, more specifically prostate cancer, using the stereoisomers of the formula (I), the salts, hydrates, prodrugs thereof and pharmaceutically acceptable esters; wherein the stereoisomers are in their individual separate forms of racemic mixtures or non-racemic mixtures with diastereoisomer excess in any proportions.

Said uses and treatment methods described above comprise the supply of a therapeutically effective amount of the compounds and their respective salts, solvates, prodrugs, stereoisomers and racemic mixtures of the same.

Another object of the present patent application is the process for the production of stereoisomers of the compound 3- (benzo [d] [1,3] dioxol-5-yl) -7- (1-hydroxypropan-2-yl) -1- (1*H*-indole-3-yl) -6, 7-dihydro-3H-oxazole [3, 4-a] pyrazine-5, 8-dione of formula (I), as described in the above schemes and the following examples.

### Examples:

It must be understood that the examples and embodiments described in detail herein illustrate the present patent application without, however, limiting the scope thereof.

### Example 1: Preparation of the Compound of formula (VIII)

In a 500 mL balloon with mechanical stirrer, addition funnel and drying tube containing CaCl2, there were added the intermediary compound (E) -methyl 2- ((benzo [d] [1, 3] dioxol-5-yl methylene) amine) -3- (1*H*-indole-3-yl) - 3-oxopropanoate of formula (A) (5, 0 g, 13,7 mmol), dry THF (90 mL) and dry pyridine (5,5 mL) which was stirred for 30 minutes. To this suspension was added, slowly, approximately 3 h, a solution of chloroacetyl chloride (1.5 mL, 19.2 mmol) in dry THF (23 mL) . After the addition, the reaction mixture was reacted for a period of 4 h at room temperature. At the end of this period, (R) -2-aminopropane-1-ol (23.7 g, 315.1 mmol) was added to the medium, and the mixture was stirred for a period of 16 h. At the end of this time, 100 mL ethanol was added. The system was submitted to a vacuum distillation, and the excess solvent removed up to the precipitation of the product. The balloon was cooled to room temperature and subsequently between 0 and 5 °C, and stirred for 2 h at this temperature. The solid was placed in a 100 mL balloon and 30 mL of ethanol was added; the mixture was stirred for a period of 30 minutes. Upon conclusion of this period, the solid was filtered and vacuum dried, leading to the forming of the compound of formula (VIII), constituted by a mixture of the compounds (VI) and (VII) corresponding to 41% yield.

The compound obtained by this method presents IUPAC nomenclature (R) -3- (benzo [d] [1 , 3] dioxol-5-yl) -7- ((R) -1-hydroxypropan-2-yl) -1- (1*H*-indole-3-yl) -6, 7-dihydro-3H-oxazole [3, 4-a] pyrazine-5, 8-dione **with** (S)-3-(benzo [d] [1,3] dioxol-5-yl) -7- ((R) -1-hydroxypropan-2-yl) -1- (1*H*-indole-3-yl) - 6, 7-dihydro-3H-oxazole [3, 4-a]pyrazine-5, 8-dione (**BL-380**), represented by the chemical structure (VIII): 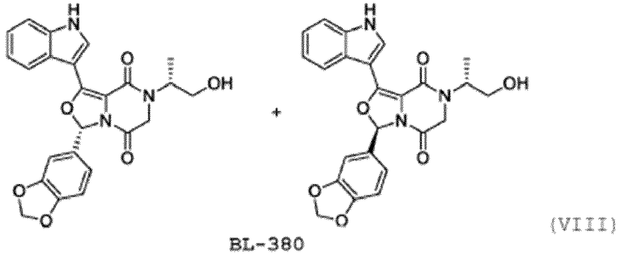 and presents the following characteristics: yellow solid; fusion point 256-262 °C; **NMR ¹H** (500 MHz, DMSO-d6) : δ 11.84 (ls, 2H) , 8.97-8.95 (m, 2H) , 7.87-7.85 (m, 2H) , 7.47-7.46 (m, 2H) , 7.17-7.09 (m, 8H) , 7.08-7.04 (m, 2H),6.04 (s, 4H) , 4.84 (t, J= 5.8 Hz, 1H) , 4.80 (t, J= 5.8 Hz, 1H) , 4.67-4.60 (m, 2H) , 4.14-4.03 (m, 4H) , 3.57-3.42 (m, 4H) , 1.11 (d, J= 7.0 Hz, 3H) , 1.08 (d, J= 7.0 Hz, 3H) ;NMR ¹³C (125 MHz, DMSO-d6) : δ 159.0, 158.7, 157.0, 156.7, 148,3, 147.6, 147.57, 147,55, 135.77, 135.76, 131.1 131.0, 130.9, 130.8, 125.2, 122.1, 121.3, 120.50, 120.49, 120.44, 120.36, 112.1, 108.3, 108.2, 106.5, 106.4, 105.2, 105.1, 102.3, 101.4, 90.3, 90.1, 61.8, 61.4, 49.4, 49.3, 46.3, 46.1, 13.2, 13.1. **HRMS** (EI) m/z calculated for C₂₄H₂₁N₃O₆: [447.1430] + found: 448.1542 [M+H] +.

The following compounds were used as intermediary reactions: (E) -methyl 2- ((benzo [d] [1, 3] dioxol-5-yl methylene) amine) -3- (1*H*-indole-3-yl) -3-oxopropanoate, described by the chemical structure (A); the compound (R) -2-aminopropane-1- ol (3), and the 2-chloroacetyl chloride (1), as exemplified below and in scheme 1.

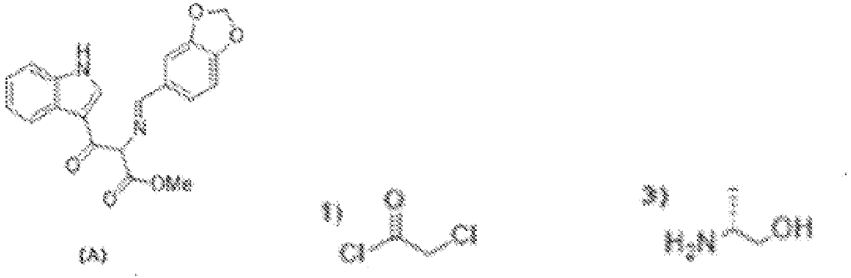

### Example 2: Preparation of the Compound of Formula (IX)

In a 500 mL balloon with mechanical stirrer, addition funnel and drying tube containing CaCl2, there were added the intermediary compound (E) -methyl 2- ((benzo [d] [1, 3] dioxol-5-yl methylene) amine) -3- (1*H*-indole-3-yl) - 3-oxopropanoate of formula (A) (5.0 g, 13.7 mmol), dry THF (90 mL) and dry pyridine (5.5 mL) which was stirred for 30 minutes. To this suspension was added, slowly, approximately 3 h, a solution of chloroacetyl chloride (1.5 mL, 19.2 mmol) in dry THF (23 mL) . After the addition, the reaction mixture was reacted for a period of 4 h at room temperature. At the end of this period, (R) -2-aminopropane-1-ol (23.7 g, 315.1 mmol) was added to the medium, and the mixture was stirred for a period of 16 h. At the end of this time, 100 mL ethanol was added. The system was submitted to a vacuum distillation, and the excess solvent removed up to the precipitation of the product. The balloon was cooled to room temperature and subsequently between 0 and 5 °C, and stirred for 2 h at this temperature. The solid was placed in a 100 mL balloon and 30 mL of ethanol was added; the mixture was stirred for a period of 30 minutes. Upon conclusion of this period, the solid was filtered and vacuum dried, leading to the forming of the compound of formula (IX), constituted by a mixture of the compounds (IV) and (V) corresponding to 40% yield.

The compound obtained by this process presents IUPAC nomenclature (R) -3- (benzo [d] [1 , 3] dioxol-5-yl) -7- ((S) -1-hydroxypropan-2-yl) -1- (1*H*-indole-3-yl) -6, 7-dihydro-3H-oxazole [3, 4-a] pyrazine-5, 8-dione **with** (S)-3- (benzo [d] [1,3] dioxol-5-yl) -7- ((S) -1-hydroxypropan-2-yl) -1- (1*H*-indole-3-yl) - 6, 7-dihydro-3H-oxazole [3, 4-a]pyrazine-5, 8-dione (**BL-241**), represented by the chemical structure (IX) : 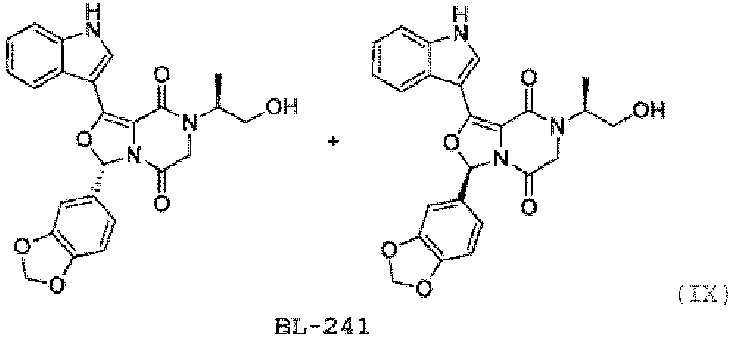 and presents the following characteristics: yellow solid; fusion point 256-261 °C; **NMR** ¹**H** (500 MHz, DMSO-d6) : δ 11.84 (is, 2H) , 8.97-8.95 (m, 2H) , 7.88-7.85 (m, 2H) , 7.47-7.46 (m, 2H) , 7.17-7.05 (m, 10H) , 6.98-6.96 (m, 2H) , 6.04 (s, 4H) , 4.84 (t, J= 5.5 Hz, 1H) , 4.80 (t, J= 5.5 Hz, 1H) , 4.67-4.60 (m, 2H) , 4.14-4.03 (m, 4H) , 3.57-3.42 (m, 4H) , 1.11 (d, J= 7.0 Hz, 3H) , 1.08 (d, J= 7.0 Hz, 3H) ; **NMR ¹³C** (125 MHz, DMSO-d6): δ 159.0, 158.7, 157.0, 156.8, 148,3, 147.6, 147.57, 147,55, 135.8, 135.78, 135.77, 131.1 131.0, 130.9, 130.8, 125.2, 122.1, 121.3, 120.5, 120.49, 120.44, 120.36, 112.1, 108.3, 108.2, 106.5, 106.4, 105.2, 105.1, 102.3, 101.4, 90.3, 90.1, 61.8, 61.4, 49.4, 49.3, 46.3, 46.1, 13.2, 13.1. **HRMS** (EI) m/z calculated for C₂₄H₂₁N₃O₆: [447.1430] + found: 448.1541 [M+H] +.

The following compounds were used as intermediary reactions: (E) -methyl 2- ((benzo [d] [1, 3] dioxol-5-yl methylene) amine) -3- (1*H*-indole-3-yl) -3-oxopropanoate, described by the chemical structure (A); and the compound (S) -2-aminopropane-1-ol (4) and the chloroacetyl chloride (1) as exemplified below and in scheme 2 .

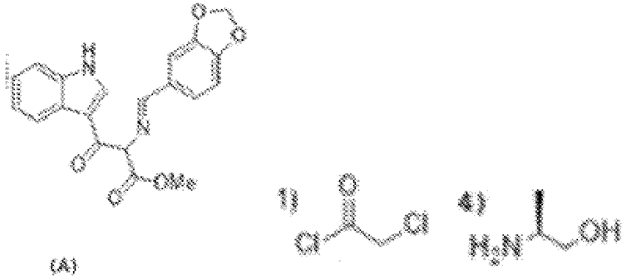

### Example 3: Process of Purification of the Diastereoisomers of Formulas (IV) and (V)

In a 1,0 L balloon there were added 4.5 g of the compound of formula (IX) (R) -3- (benzo [d] [1, 3] dioxol-5-yl) -7- ((S) -1- hydroxypropan-2-yl) -1- (1*H-*indole-3-yl) -6, 7-dihydro-3H- oxazole [3, 4-a] pyrazine-5, 8-dione **with** (S)-3- (benzo [d] [1,3] dioxol-5-yl) -7- ((S) -1-hydroxypropan-2-yl) -1- (1*H*-indole-3-yl) - 6, 7-dihydro-3H-oxazole [3, 4-a]pyrazine-5, 8- dione (BL-241), and 75 mL of a mixture of THF/H₂O (8:2). The suspension was heated at 50°C up to the solubilization. After the solubilization, 560 mL of methanol was added to the medium and stirred for 2 hours at room temperature. The solid formed was vacuum filtered, washed in 50 mL of methanol, and kiln dried at 100°C for 4 hours. This step generated the isolation of one of the compounds BL-236A or BL-236B, which presents the following characteristics: yellow solid, fusion point: 227-231°C; **NMR** ¹**H** (300 MHz , DMS0-d6) : δ 11.89 (ls, 1H) , 8.97 (d, J= 1.8 Hz, 1H) , 7.86 (d, J= 7.9 Hz, 1H) , 7.46 (d, J= 7.9 Hz, 1H) , 7.18-7.03 (m, 5H) , 6.98 (dd, J= 7.4 Hz, J= 1.0 Hz, 1H) , 6.04 (s, 2H) , 4.88 (t, J= 5.5 Hz, 1H) , 4.69-4.58 (m, 1H) , 4.07 (dd, J= 22.5 Hz, J= 17.5 Hz, 2H) , 3.58-3.41 (m, 2H) , 1.07 (d, J= 6.9 Hz, 3H) ; **NMR** ¹³**C** (75 MHz, DMSO-d6) : δ 159.9, 157.1, 148.4, 147.7, 147.6, 135.8, 131.2, 130.9, 125.3, 122.2, 121.4, 120.6, 120.5, 112.3, 108.4, 106.6, 105.2, 102.5, 101.5, 90.2, 61.4, 49.4, 46.1, 13.2; **HRMS** (EI) m/ z calculated for C₂₄H₂₁N₃O₆: [447.1430]+, found: 448.1321 [M+H]+. The chemical structure of the compound was elucidated by nuclear magnetic resonance analysis, high resolution mass and X-ray crystallography.

The mother liquor of the previous filtration was returned to the reaction balloon and 200 mL of H2O were slowly added, leading to turbidity of the reaction medium, which was stirred for one hour at room temperature. The suspension was vacuum filtered and washed with 50 mL ethanol. The solid obtained was resuspended in 50 mL of ethanol and filtered once more, which was subsequently kiln dried at 100°C for 4 hours, leading to the isolation of the other compound of BL-236), which presents the following characteristics: yellow solid, fusion point: 221-225°C; **NMR** ¹**H** (300 MHz , DMSO-d6) : δ 11.90 (ls, 1H) , 8.96 (s, 1H) , 7.85 (d, J= 7.9 Hz, 1H) , 7.46 (d, J= 7.9 Hz, 1H) , 7.18-7.03 (m, 5H) , 6.97 (d, J= 7.9 Hz, 1H) , 6.04 (s, 2H) , 4.83 (t, J= 5.7 Hz, 1H) , 4.67-4.60 (m, 1H) , 4.09 (dd, J= 25.7 Hz, J= 17.6 Hz, 2H) , 3.56-3.41 (m, 2H) , 1.10 (d, J= 6.9 Hz, 3H) ; **NMR** ¹³**C** (75 MHz, DMSO-d6) : δ 158.7, 156.8, 148.4, 147.6 (2C) , 135.8, 131.1, 130.9, 125.3, 122.2, 121.4, 120.6, 120.5, 112.2, 108.3, 106.6, 105.2, 102.4, 101.5, 90.4, 61.8, 49.4, 46.3, 13.3; **HRMS** (EI) m/ z calculated for C₂₄H₂₁N₃O₆: [447.1430]+, found: 448.1530 [M+H]+. The chemical structure of the compound was elucidated by nuclear magnetic resonance analysis, high resolution mass and X-ray crystallography.

With this process we have the separation of the compounds (S) 3- (benzo [d] [1,3] dioxol-5-yl) -7- ((S) -1-hydroxypropan-2-yl) -1- (1*H*-indole-3-yl) - 6, 7-dihydro-3H-oxazole [3, 4-a]pyrazine-5, 8-dione **(BL-236A)** and (R) - 3- (benzo [d] [1, 3] dioxol-5-yl)-7- ((57)- 1- hydroxypropan-2-yl) -1- (1*H*-indole-3-yl) -6, 7-dihydro-3H-oxazole [3, 4-a] pyrazine-5, 8-dione (**BL-236B**)**,** represented by the structures (IV) and (V), respectively.

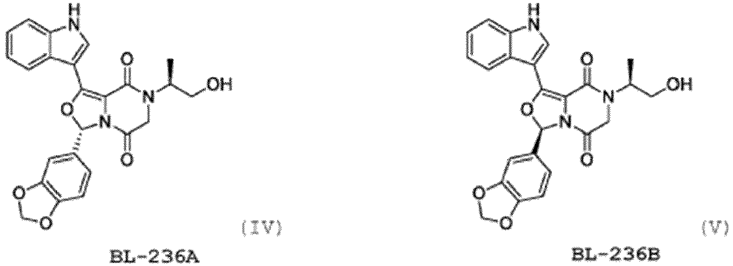

### Example 4: Process for Purification of the Diastereoisomers of Formulas (VI) and (VII)

In a 1,0 L balloon there were added 4.5 g of the compound of formula (VIII) (R) -3- (benzo [d] [1, 3] dioxol-5-yl) -7- ((R) -1- hydroxypropan-2-yl) -1- (1*H-*indole-3-yl) -6, 7-dihydro-3*H*- oxazole [3, 4-a] pyrazine-5, 8-dione **with** (S)-3- (benzo [d] [1,3] dioxol-5-yl) -7- ((R) -1-hydroxypropan-2-yl) -1- (1*H*- indole-3- yl) - 6, 7-dihydro-3*H*-oxazole [3, 4-a]pyrazine-5, 8- dione (**BL-380**), and 75 mL of a mixture of THF/H₂O (8:2). The suspension was heated at 50°C up to the solubilization. After the solubilization, 560 mL of methanol was added to the medium and stirred for 2 hours at room temperature. The solid formed was vacuum filtered, washed in 50 mL of methanol, and kiln dried at 100°C for 4 hours. This step generated the isolation of one of the compounds BL-236A or BL-236B, which presents the following characteristics: yellow solid, fusion point: 233- 236 ° C ; **NMR** ¹**H** (300 MHz , DMSO-d6) : δ 11.89 (ls, 1H) , 8.97 (d, J= 2.4 Hz, 1H) , 7.86 (d, J= 7.9 Hz, 1H) , 7.46 (d, J= 8.0 Hz, 1H) , 7.18-7.04 (m, 5H) , 6.98 (d, J= 8.3 Hz, 1H) , 6.04 (s, 2H) , 4.88 (t, J= 5.7 Hz, 1H) , 4.67-4.60 (m, 1H) , 4.07 (dd, J= 22.5 Hz, J= 17.5 Hz, 2H) , 3.58-3.41 (m, 2H) , 1.06 (d, J= 6.9 Hz, 3H) ; **NMR** ¹³**C** (75 MHz, DMSO-d6) : δ 159.1, 157.1, 148.4, 147.7, 147.6, 135.8, 131.2, 130.9, 125.3, 122.2, 121.4, 120.6, 120.5, 112.3, 108.4, 106.6, 105.2, 102.4, 101.5, 90.2, 61.4, 49.4, 46.1, 13.2; **HRMS** (EI) m/ z calculated for C₂₄H₂₁N₃O₆: [447.1430]+, found: 448.1540 [M+H]+. The chemical structure of the compound was elucidated by nuclear magnetic resonance analysis, high resolution mass and X-ray crystallography

The mother liquor of the previous filtration was returned to the reaction balloon and 200 mL of H2O were added slowly, leading to turbidity of the reaction medium, which was stirred for one hour at room temperature. The suspension was vacuum filtered and washed with 50 mL ethanol. The solid obtained was resuspended in 50 mL of ethanol and filtered once more, which was subsequently kiln dried at 100°C for 4 hours, leading to the product of interest (another BL-239 molecule) Yellow solid, fusion point: 223-225 ° C ; NMR **¹**H (300 MHz , DMSO-d6) : δ 11.90 (ls, 1H) , 8.96 (s, 1H) , 7.85 (d, J= 8.2 Hz, 1H) , 7.47 (d, J= 8.2 Hz, 1H) , 7.18- 7.03 (m, 5H) , 6.98 (d, J= 8.0 Hz, 1H) , 6.04 (s, 2H) , 4.84 (t, J= 5.6 Hz, 1H) , 4.69-4.58 (m, 1H) , 4.09 (dd, J= 25.7 Hz, J= 17.6 Hz, 2H) , 3.55-3.41 (m, 2H) , 1.10 (d, J= 6.7 Hz, 3H) ; **NMR ¹³C** (75 MHz, DMSO-d6) : δ 158.7, 156.8, 148.4, 147.6 (2C), 135.8, 131.1, 130.9, 125.3, 122.2, 121.4, 120.6, 120.5, 112.2, 108.3, 106.6, 105.2, 102.4, 101.5, 90.4, 61.8, 49.4, 46.3, 13.3; **HRMS** (EI) m/z calculated for C₂₄H₂₁N₃O₆: [447.1430]+, found: 448.1529, [M+H]+. The chemical structure of the compound was elucidated by nuclear magnetic resonance analysis, high resolution mass and X-ray crystallography.

With this process we have the isolation of the compounds (S)-3-(benzo[d] [1,3]dioxol-5-yl)-7-((R) -1-hydroxypropan-2-yl) -1-(1*H*-indole-3-yl ) - 6, 7-dihydro-3H-oxazole[3, 4-a]pyrazine-5, 8-dione (BL-239A) and (R) -3-(benzo [d] [1 , 3] dioxol-5-yl) -7- ((R) -1-hydroxypropan-2-yl) -1- (1*H*-indole-3-yl) -6, 7-dihydro-3H-oxazole [3, 4-a] pyrazine-5, 8-dione (BL-239B), represented by the structures (VI) and (VII) respectively.

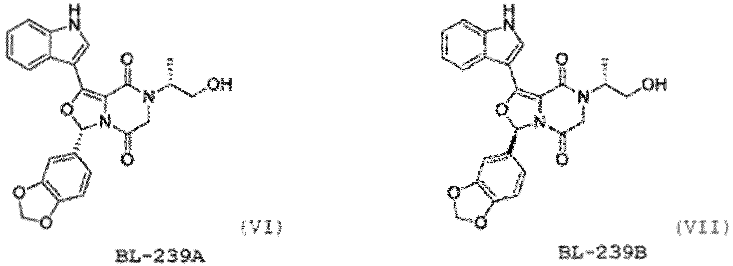

### Example 7: In vitro evaluation of the Inhibitory Potential of the Compounds under the Activity of the Human Phosphodiesterase Enzyme 5.

The evaluation of the compound is studied using BPS Bioscience enzyme assay kit (catalogue 60350). The PDE5A1 Assay kit is designed for identification of PDE5A1 inhibitors using fluorescence polarization. The assay is based on the binding of a fluorescent nucleotide monophosphate generated by PDE5A1 enzyme to the binding agent.

In this methodology only two simple steps on a microtiter plate are required for PDE5A1 reactions. First, the fluorescently labeled cGMP is incubated with PDE5A1 for 1 hour. Second, the binding agent is added to the reaction mixture to produce a change in fluorescent polarization that can then be measured using a fluorescence reader equipped for the measurement of fluorescence polarization.

For the assay the storage reagent FAM-Cyclic-3',5'-GMP (20µM) is diluted with PDE assay buffer to achieve the solution with a 200 µM concentration; the PDE5A1 enzyme is thawed and diluted with the PDE buffer obtaining a PDE5A1 solution at 10 pg/mL.

The assay compounds are diluted with DMSO 100% (v/v) at a concentration of 1000 µM and subsequently diluted in a Sodium chloride 0.9% solution to obtain a curve of concentrations of interest for the assay. The compounds are diluted in concentrations of: 10 nM, 30 nM, 50 nM, 100 nM, 300 nM, 500 nM, 1 µM, 3 µM, 5 µM (DMSO 10% v/v) .

The study is comprised of groups: (i) substrate control; (ii) positive control (KIT); (iii) samples in several concentrations; (iv) tadalafil (comparative control). Each parameter (group) is triple labeled.

### Assay Procedure:

- For the 4 groups there is added, to each microplate well, 25 mL of the FAM-Cyclic-3' reagent, 5' -GMP (200 µM).
- To the substrate control group there is added, to each well, 5 mL of vehicle (DMSO 10% v/v) and 20 mL PDE Buffer.
- To the positive control group there is added, to each well, 5 mL of vehicle (DMSO 10% v/v).
- To the tadalafil group: there is added 5 mL of the solution in the concentrations of interest.
- To the test group there is added 5 mL of the sample solution (test compound)

### OBSERVATION: the parameters tested are in triplicate.

To begin the reaction, add 20 mL of the PDE5A1 enzyme (diluted as described above) to the microplate wells designated as Positive Control and Samples (test and tadalafil), and incubate the microplate, for 1 hour, at a temperature of 25°C.

After the incubation period, the reaction is interrupted by the addition, in all the wells, of 100 mL of the binding agent solution and the microplate is incubated for 30 minutes, under light stirring, at room temperature. The reading of the microplate is carried out in Polarized Fluorescence (475-495 nm - excitation and 518-538 nm - emission detection) 500 nanoseconds integration time.

For the compound BL-380 there were prepared the concentrations of 10 nM, 30 nM, 50 nM, 100 nM, 300 nM, 500 nM, 1 µM (1000 nM) , 3 µM (3000 nM) and 5 µM (5000 nM) . Based on the results obtained, (table 1) it is possible to observe that the compound BL-380 was capable of inhibiting the PDE5 in a dose-dependent manner, however, this inhibition was only observed as from the concentration of 300 nM of BL-380.

**Table 1- Inhibition Percentage of the PDE5 Enzyme by Compound BL-380.**

| **Compounds** | **% of Inhibition (PDE5A1)** |
|---|---|
| BL-380 10 nM | 0 |
| BL-380 30 nM | 0 |
| BL-380 50 nM | 0 |
| BL-380 100 nM | 0 |
| BL-380 300 nM | 15 ± 3 |
| BL-380 500 nM | 25 ± 3 |
| BL-380 1000 nM | 55 ± 4 |
| BL-380 3000 nM | 65 ± 2 |
| BL-380 5000 nM | 78 ± 1 |
| **Tadalafyl 5000 nM** | 96 ± 2 |

For the compound BL-241 there were prepared concentrations of 500 nM, 1 µM (1000 nM) and 3 µM (3000 nM) . Based on the results obtained (table 2) it is possible to observe that the compound BL-241 was also capable of inhibiting the PDE5 in a dose-dependent manner.

**TABLE 2 - Inhibition Percentage of the PDE5 Enzyme by Compound BL-241.**

| Compounds | % of Inhibition (PDE5A1) |
|---|---|
| BL-241 500 nM | 57% |
| BL-241 1000 nM | 71% |
| BL-241 3000 nM | 88% |

### Example 6: Evaluation of the Antiproliferative Effect of the Compounds in Cultures of Human Cell Lineages of Prostate, Gastric and Bladder Cancer.

For the execution of the assay, the compounds are diluted in DMSO 100% (v/v) at a concentration of 1000 µM and subsequently diluted in a Sodium Chloride solution 0.9% to obtain the storage solutions of interest for use in the assays. The final concentration of interest will be directly diluted in the cell culture medium.

For the assay there are used the following tumor cell lineages: PC3-human prostate cancer cells; LNCAPhuman prostate cancer cells; DU145-human prostate cancer; MNK45-human gastric cancer and RT4-human bladder cancer cells.

The tumor cell lineages are maintained in culture according to standard procedures established by the Cell Bank of Rio de Janeiro (lineage place of origin). Procedure:
- For the assay, the tumor cells are seeded in the concentration of 10.000 cells per well (final volume of the reaction of 200. mL), the plate is incubated for 8 hours, for the cells to adhere to the wall of the wells. After this period, the plate is divided in groups:
   (a) test group: 20 mL of the samples were added (test compound) in the concentrations of interest;
   (b) positive control group: 20 mL of culture medium were added (100% of cell proliferation);
   (c) negative control group: 200 mL of culture medium and 20 mL of the vehicle used were added to dilute the compounds (without cells - zero proliferation);
   (d) control group: 20 mL were added of the vehicle used to dilute the compounds. This group is important to evaluate whether the vehicle used interferes in the cell proliferation.
      OBSERVATION: the treatment with the concentrations of samples (test compounds) and of the controls are carried out in triplicate.
- Incubate the plate for 72 hours in a CO₂ kiln at 37°C.
- After the incubation period, add 100mL /well of the solution with DNA marker from the CYQuant Kit and incubate the plate, for 60 minutes, in a CO₂ incubator at 37°C. The fluorescence intensity is determined using a fluorescence microplate reader with 485 nm excitation and 530 nm emission detection. The results obtained were normalized relative to the positive control group (100% proliferation).

For the compounds there are prepared the storage solutions in the concentrations of: 100 nM, 1 µM, 3 uM, 5 µM, 10 µM and 30 µM (DMSO 10% v/v) . While the final concentrations in the test are of: 10 nM, 100 nM, 300 nM, 500 nM, 1000 nM and 3000 nM (in DMSO 1% v/v).

The results obtained demonstrate that the BL-380 compound presented antiproliferative effect for the 3 prostate cancer tumor lineages tested PC3, LNCAP and DU145) and for the bladder cancer tumor lineage (RT4), as can be observed in table 3. And the BL-241 compounds presented antiproliferative effect for the 3 prostate cancer tumor lineages tested (PC3, LNCAP and DU145) and for the bladder cancer tumor lineage (RT4), as can be observed in table 4.

**TABLE 3 - Percentage of Cell Proliferation Inhibition - BL-380 Compound.**

| Concentration of compound BL-380 | % Cell Proliferation Inhibition (intracellular DNA) | | | | |
|---|---|---|---|---|---|
| | PC-3 (average ± E.P.M.) | LNCAP (average + E.P.M.) | DU145 (average + E.P.M.) | MNK45 (average ± E.P.M.) | RT4 (average ± E.P.M.) |
| 10 nM | 39 ± 6 | 26 ± 10 | - | - | - |
| 100 nM | 25 ± 5 | 12 ± 3 | 12 ± 4 | 7 ± 2 | 8 ± 6 |
| 300 nM | 28 ± 2 | 29 ± 6 | 10 ± 5 | 0 | 5 ± 5 |
| 500 nM | 48 ± 6 | 27 ± 2 | 12 ± 2 | 0 | 3 ± 5 |
| 1000 nM | 40 ± 3 | 37 ± 2 | 10 ± 3 | 0 | 3 ± 1 |
| 3000 nM | 54 ± 4 | 48 ± 6 | 31 ± 5 | 16 ± 5 | 58 ± 4 |

**Table 4 - Percentage of Cell Proliferation Inhibition - Compound BL-241**

| Concentration of compound BL-241 | % Cell Proliferation Inhibition (intracellular DNA) | | | | |
|---|---|---|---|---|---|
| | PC-3(average ± E.P.M.) | LNCAP (average ± E.P.M.) | DU145(average ± E.P.M.) | MNK45(average ± E.P.M.) | RT4 (average ± E.P.M.) |
| 10 nM | 27 ± 11 | 25 ± 2 | - | - | - |
| 100 nM | 36 ± 7 | 37 ± 3 | 33 ± 8 | 25 ± 1 | 28 ± 6 |
| 300 nM | 42 ± 6 | 38 ± 4 | 17 ± 3 | 10 ± 2 | 5 ± 2 |
| 500 nM | 46 ± 1 | 44 ± 2 | 18 ± 2 | 2 ± 1 | 4 ± 2 |
| 1000 nM | 33 ± 3 | 34 ± 6 | 22 ± 2 | 4 ± 1 | 11 ± 8 |
| 3000 nM | - | - | 43 ± 5 | 20 ± 3 | 57 ± 4 |

## Claims

1. Compound **characterized by** being 3- (benzo [d] [1, 3] dioxol-5-yl) -7- (1-hydroxypropan-2-yl) -1- (1*H*-indole-3-yl) -6, 7-dihydro-3H-oxazole [3, 4-a] pyrazine-5, 8-dione of formula (I), or the stereoisomers, salts, solvates, hydrates, prodrugs and pharmaceutically acceptable esters thereof

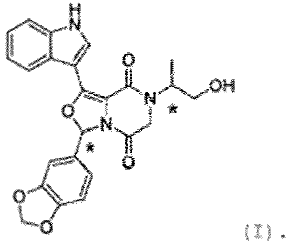

2. Compound, according to claim 1, **characterized by** the fact that the stereoisomers are in their individual separate forms and/or in the forms of racemic mixtures or non-racemic mixtures with diastereoisomer excess in any proportions.

3. Compound, according to claim 1, **characterized by** being 3- (benzo [d] [1, 3] dioxol-5-yl) -7- ((S) -1-hydroxypropan-2-yl) -1- (1*H*-indole-3-yl) - 6, 7-dihydro-3H-oxazole [3, 4-a] pyrazine-5, 8-dione (BL-236), represented by the formula (II):

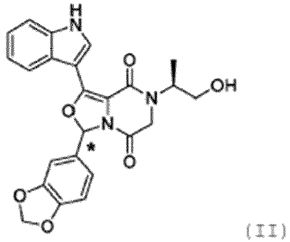

4. Compound, according to claim 1, **characterized by** being 3- (benzo [d] [1, 3] dioxol-5-yl) -7- ((R) -1-hydroxypropan-2-yl) -1- (1*H*-indole-3-yl) - 6, 7-dihydro-3H-oxazole [3, 4-a] pyrazine-5, 8-dione (BL-239), represented by formula (III):

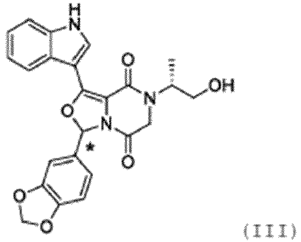

5. Compound, according to claim 3, **characterized by** being (S) -3- (benzo [d] [1, 3] dioxol-5-yl) -7- ((S) -1-hydroxypropan-2-yl) -1- (1*H*-indole-3-yl) -6, 7-dihydro-3H-oxazole [3, 4-a] pyrazine-5, 8-dione (BL-236A), represented by formula (IV):

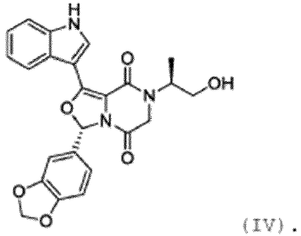

6. Compound, according to claim 3, **characterized by** being (R) - 3- (benzo [d] [1,3] dioxol-5-yl) - 7- ((S) -1-hydroxypropan-2-yl) -1- (1*H*-indole-3-yl) -6, 7-dihydro-3H-oxazole [3, 4-a] pyrazine-5, 8-dione (BL-236B), represented by formula (V):

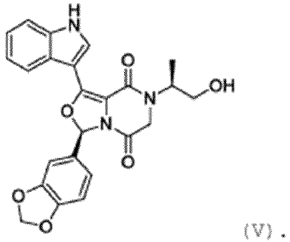

7. Compound, according to claim 4, **characterized by** being (S) -3- (benzo [d] [1,3] dioxol-5-yl) -7- ((R) -1-hydroxypropan-2-yl) -1- (1*H*-indole-3-yl) -6, 7-dihydro-3H-oxazole [3, 4-a] pyrazine-5, 8-dione (BL-239A), represented by formula (VI):

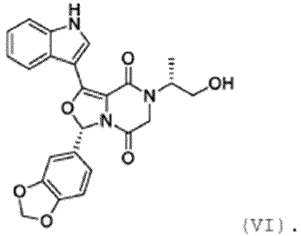

8. Compound, according to claim 4, **characterized by** being (R) -3- (benzo [d] [1,3] dioxol-5-yl) -7- ((R) -1-hydroxypropan-2-yl) -1- (1*H*-indole-3-yl) -6, 7-dihydro-3H-oxazole [3, 4-a] pyrazine-5, 8-dione (BL-239B), represented by formula (VII):

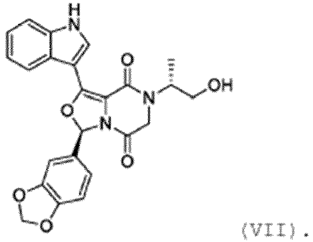

9. Compound, according to claim 2, **characterized by** being a mixture of diastereoisomers of 3- (benzo[d] [1,3] dioxol-5-yl)-7-(1-hydroxypropan-2-yl) - 1- (1*H*-indole-3-yl) - 6, 7-dihydro-3H-oxazole [3, 4-a] pyrazine-5, 8 -dione.

10. Compound, according to claim 9, **characterized by** being a mixture of (R) -3- (benzo [d] [1 , 3] dioxol-5-yl) -7- ((R) -1-hydroxypropan-2-yl) - 1- (1*H*-indole-3-yl) - 6, 7-dihydro-3H-oxazole [3, 4-a] pyrazine-5, 8-dione with (S)-3- (benzo [d] [1,3] dioxol-5-yl) -7- ((R) -1-hydroxypropan-2-yl) -1-(1*H*- indo 1-3- yl) - 6, 7-dihydro-3H-oxazole [3, 4-a]pyrazine-5, 8-dione (BL-380), represented by formula (VIII):

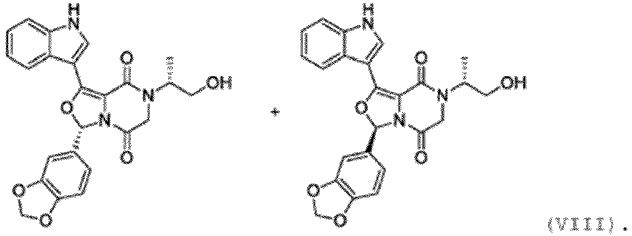

11. Compound, according to claim 9, **characterized by** being a mixture of (R) -3- (benzo [d] [1 , 3] dioxol-5-yl) -7- ((S)-1-hydroxypropan-2-yl) - 1- (1*H*-indole-3-yl) - 6, 7-dihydro-3H-oxazole [3, 4-a] pyrazine-5, 8-dione with (S)-3-(benzo [d] [1,3] dioxol-5-yl) -7- ((S) -1-hydroxypropan-2-yl) - 1- (1*H*-indole-3-yl ) - 6, 7-dihydro-3H-oxazole [3, 4-a]pyrazine-5, 8-dione (BL-241), represented by formula (IX) :

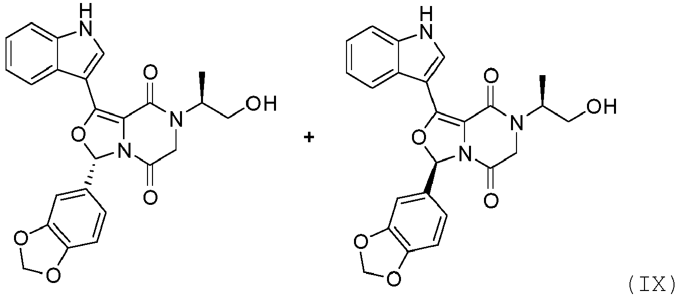

12. Mixture of stereoisomers as defined in any one of claims 2 to 11 **characterized by** the fact that it is a racemic mixture or a non-racemic mixture.

13. Mixture of stereoisomers, according to claim 12, **characterized by** the fact that the mixtures are non-racemic, with diastereoisomer excess in any proportions.

14. Mixture of stereoisomers, according to claim 13, **characterized by** the fact that the diastereoisomeric proportion is in the range from 1:99 to 99:1.

15. Mixture of stereoisomers, according to claim 13, **characterized by** the fact that the diastereoisomeric proportion is in the range from 1:2 to 2:1.

16. Mixture of stereoisomers, according to claim 13, **characterized by** the fact that the diastereoisomeric proportion is preferably 1:1.

17. Mixture of stereoisomers, according to claim 12, **characterized by** being a mixture of (R)-3- (benzo [d] [1,3] dioxol-5-yl) -7- ((R) -1-hydroxypropan-2-yl) -1-(1*H*-indole-3-yl) - 6, 7-dihydro-3H-oxazole [3, 4-a]pyrazine-5, 8- dione with (S) -3- (benzo [d] [1, 3] dioxol-5-yl) -7- ((R) -1-hydroxypropan-2-yl) -1- (1*H*-indole-3-yl) -6, 7-dihydro-3H-oxazole [3, 4-a] pyrazine-5, 8-dione (BL-380), represented by formula (VIII): 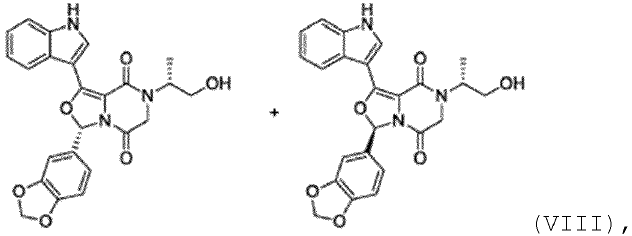 wherein the diastereoisomeric proportion is in the range from 1:99 a 99:1.

18. Mixture of stereoisomers, according to claim 12, **characterized by** being a mixture of (R) -3- (benzo [d] [1,3]dioxol- 5-yl) -7- ((S) -1-hydroxypropan-2-yl) -1- (1*H-*indole-3-yl) -6, 7-dihydro-3H-oxazole [3 , 4-a] pyrazine-5, 8-dione with (S) -3- (benzo [d] [1,3] dioxol-5-yl) -7- ((S) -1-hydroxypropan-2-yl) - 1- (1*H*-indole-3-yl) - 6, 7-dihydro-3H-oxazole [3, 4-a]pyrazine-5, 8-dione (BL-241), represented by formula (IX): 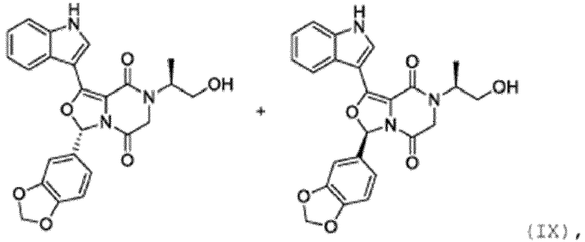 wherein the diastereoisomeric proportion is in the range from 1:99 a 99:1.

19. PHARMACEUTICAL COMPOSITION **characterized by** comprising a therapeutically effective amount of one or more compounds described in any one of claims 1 to 11, or mixtures of the same as defined in claims 12 to 18, and one or more pharmaceutically acceptable excipients.

20. PHARMACEUTICAL COMPOSITION, according to claim 19, **characterized by** comprising as active ingredient at least one of the following stereoisomers selected from the group constituted by:
(S)-3-(benzo[d] [1,3] dioxol-5-yl) -7- ((S) -1-hydroxypropan-2-yl) -1- (1*H*-indole-3-yl) - 6, 7-dihydro-3H-oxazole [3, 4-a] pyrazine-5, 8-dione (**BL-236A**);
(R)-3-(benzo[d] [1,3] dioxol-5-yl) -7- ((S) -1-hydroxypropan-2-yl) -1- (1*H*-indole-3-yl) - 6, 7-dihydro-3H-oxazole [3, 4-a] pyrazine-5, 8-dione (**BL-236B**);
(S)-3-(benzo[d] [1,3] dioxol-5-yl) -7- ((R) -1-hydroxypropan-2-yl) -1- (1*H*-indole-3-yl) - 6, 7-dihydro-3H-oxazole [3, 4-a] pyrazine-5, 8-dione (**BL-239A**); and
(R)-3-(benzo[d] [1,3] dioxol-5-yl) -7- ((R) -1-hydroxypropan-2-yl) -1- (1*H*-indole-3-yl) - 6, 7-dihydro-3H-oxazole [3, 4-a] pyrazine-5, 8-dione (**BL-239B**);
their pharmaceutically acceptable salts, solvates, hydrates, prodrugs and esters; wherein the stereoisomers are in their individual separate forms and/or in the forms of racemic mixtures or non-racemic mixtures with diastereoisomeric excess in any proportions; and one or more pharmaceutically acceptable excipients.

21. PHARMACEUTICAL COMPOSITION, according to claim 19, **characterized by** comprising as active ingredient at least a mixture of stereoisomers selected from the group constituted by:
(R) -3- (benzo [d] [1,3] dioxol-5-yl) -7- ((R) -1-hydroxypropan-2-yl) -1- (1*H*-indole-3-yl) - 6, 7-dihydro-3H-oxazole [3, 4-a] pyrazine- 5, 8-dione **with** (S) -3- (benzo [d] [1, 3] dioxol-5-yl) -7- ((R) -1-hydroxypropan-2-yl) -1-(1*H*-indole-3-yl) -6, 7-dihydro-3H-oxazole [3, 4-a] pyrazine-5, 8-dione (BL-380); and
(R)-3-(benzo[d] [1,3] dioxol-5-yl) -7- ((S) -1-hydroxypropan-2-yl) -1- (1*H*-indole-3-yl) - 6, 7-dihydro-3H-oxazole [3, 4-a] pyrazine-5, 8-dione **with** (S) -3- (benzo [ d] [1, 3] dioxol-5-yl) -7- ((S) -1-hydroxypropan-2-yl) -1-(1*H*-indole-3-yl) -6, 7-dihydro-3H-oxazole [3, 4-a] pyrazine-5, 8-dione (BL-241);
wherein the diastereoisomers can be in any proportions; and one or more pharmaceutically acceptable excipients.

22. Pharmaceutical composition, according to claims 19 to 21, **characterized by** being for oral, topic, injectable, nasal and rectal administration.

23. Pharmaceutical composition comprising a compound as defined in any one of claims 1 to 11, or a mixture of same, as defined in claims 12 to 18, **characterized by** being used as inhibitor of the enzymes for phosphodiesterase and antitumor.

24. Pharmaceutical composition, according to claim 19, **characterized by** being used in the treatment of benign prostatic hyperplasia, cancer, disorders and/or conditions that are treatable with tissue relaxation and disorders that are treatable with phosphodiesterase inhibitors.

25. Pharmaceutical composition, according to claim 24, **characterized by** being for the treatment of prostate cancer.

26. Pharmaceutical composition, according to claim 24, **characterized by** being for the treatment of benign prostatic hyperplasia and prostate cancer.

27. Use of the compounds as defined in any one of claims 1 to 11, or mixture as defined in claims 12 to 18, **characterized by** being for the preparation of a pharmaceutical composition for inhibition of the phosphodiesterase enzymes.

28. Use of the compounds as defined in any one of claims 1 to 11, or mixture as defined in claims 12 to 18, **characterized by** being for the preparation of an antitumor pharmaceutical composition.

29. Use of the compounds as defined in any one of claims 1 to 11, or mixture as defined in claims 12 to 18, **characterized by** being for the preparation of pharmaceutical compositions for the treatment of benign prostatic hyperplasia, cancer, disorders and/or conditions that are treatable with tissue relaxation and disorders that are treatable with phosphodiesterase inhibitors.

30. Use of the pharmaceutical composition defined in any one of claims 19 to 22, **characterized by** being for the treatment of benign prostatic hyperplasia and prostate cancer.

31. Use, according to claim 28, **characterized by** being for the treatment of prostate cancer.

32. Method for treatment or prevention of disfunctions associated with the activity of the phosphodiesterases and cancer, **characterized by** the administration of an effective amount of at least one of the compounds as defined in any one of claims 1 to 11 or mixture as defined in any one of claims 12 to 18.
32. Method, according to claim 31, **characterized by** being for the treatment and/or prophylaxis of benign prostatic hyperplasia and/or prostate cancer.

33. Method for treatment or prevention of benign prostatic hyperplasia and/or prostate cancer, **characterized by** administering an effective amount of at least one of the compounds as defined in any one of claims 1 to 11 or mixture as defined in any one of claims 12 to 18.
